# EUROPEAN PATENT APPLICATION

(11) **EP 3 026 436 A1**
(43) Date of publication of application: **01.06.2016**
(21) Application number: 14306885.6
(22) Date of filing: 26.11.2014
(51) Int. Cl.: G01N 33/68, C07K 16/00, C07K 14/00, G01N 33/74

(54) **Diagnostic of obliterative arteriopathies**

(71) Applicant: Universite d'Aix Marseille, 13284 Marseille Cedex 07 (FR); Assistance Publique Hôpitaux de Marseille, 13354 Marseille Cedex 5 (FR); INSTITUT NATIONAL DE LA SANTE ET DE LA RECHERCHE MEDICALE (INSERM), 75013 Paris (FR)
(72) Inventor: RUF, Jean, F-13190 Allauch (FR); GUIEU, Régis, F-13009 Marseille (FR); PAGANELLI, Franck, F-13008 Marseille (FR); BONELLO, Laurent, F-13320 Bouc Bel Air (FR); MOTTOLA, Giovanna, F-13011 Marseille (FR); KIPSON, Nathalie, F-83390 Cuers (FR)
(74) Representative: Gevers & Orès

(57) **Abstract**

The invention relates to a method of diagnosis or prognosis of an obliterative arteriopathy in a subject, comprising determining the expression level of adenosine A_{2A} receptors expressed at the cell surface of peripheral blood mononuclear cells obtained from said subject. The invention also relates to an agonist-like monoclonal antibody directed to the human adenosine A_{2A} receptor, useful for carrying out this method.

## Description

The present invention relates to markers for the diagnosis or prognosis of an obliterative arteriopathy, such as coronary artery disease.

Obliterative arteriopathies consist of a group of pathologies characterized by a stenosis of arteries. Such stenosis may affect any kind of arteries, particularly coronary arteries, brain arteries, lower limb arteries. The most frequent kind of obliterative arteriopathy is the coronary artery disease.

Coronary artery disease (CAD) is the major type of heart disease and cause of heart attacks. It is one of the main causes of death in the world. CAD is caused by plaque building up along the inner walls of the arteries of the heart, which narrows the arteries and reduces blood flow to the heart. Most individuals with CAD show no evidence of disease for decades as the disease progresses before the first onset of symptoms (most of the time a heart attack), finally arises.

Adenosine is an endogenous purine nucleoside that is produced by adenosine triphosphate (ATP) hydrolysis and released in the extracellular spaces during ischemia (Sollevi *et al.,* 1986; Paganelli *et al.,* 2000). It acts through four classes of G protein-coupled receptors, namely A₁, A_{2A}, A_{2B} and A₃ receptors to exert various physiological effects. Through activation of adenosine A_{2A} receptors (A_{2A}R), adenosine is a coronary vasodilator that increases coronary blood flow (Olsson, 1990; Shryock *et al.,* 1997).

A_{2A}R and to a lesser extent A_{2B}R are implicated in ischemia-induced coronary vasodilation (Eckle *et al.,* 2007, Berwick *et al.,* 2012, Sanjani *et al.,* 2011). During experimental coronary reactive hyperaemia, adenosine increases coronary blood flow up to 3 fold in a dose-dependent manner (Berwick *et al.,* 2012). This vasodilation occurs via A_{2A}R and involves Kv and K ATP channels (Berwick *et al.,* 2012).

Such coronary vasodilation constitutes therefore an adaptive response to the drop in O₂ supply that occurs during hypoxemia or ischemia (Grenz *et al.,* 2011; Hedegaard *et al.,* 2014). During exercise stress testing, myocardium O₂ demand increases, which can lead to ischemia in patients with CAD.

Deharo *et al.* (2012) have reported that low A_{2A}R expression is associated with low adenosine plasma concentration (APC) while high A_{2A}R expression is associated with high APC. Thus A_{2A}R expression partly controls *per se* extracellular APC by modulating the expression of the nucleoside transporter (Pinto-Duarte *et al.,* 2005). It was also shown that the polymorphism of A_{2A}R is associated with the infarct size in patients with ischemic cardiomyopathy (Tang *et al.,* 2007). This A_{2A}R polymorphism is associated with changes in A_{2A}R expression level (Saadjian *et al.,* 2009).

Treadmill is the most commonly used test to diagnose and evaluate coronary artery disease. However, its predictive value is limited (Nyman *et al.,* 1993). In particular electrocardiogram (ECG) change and chest pain may be lacking despite ischemic burden in some patients. Accordingly, the use of additional investigations such as stress echography, magnetic resonance imaging (MRI), and scintigraphy were proposed to overcome these limitations. However, their availability is limited and their costs are high.

The provision of a new biological marker in the diagnosis or prognosis of obliterative arteriopathy, such as coronary artery disease, is of clinical interest. This marker could further increase the predictive value of treadmill test.

The inventors have found that the expression level of A_{2A}R expressed at the cell surface of peripheral blood mononuclear cells (PBMC) in a subject constitutes a novel biological marker of coronary artery disease, especially when it is combined with the determination of the presence or the absence of A_{2A}R of reserve (spare A_{2A}R) and/or the determination of APC change during exercise stress testing (EST) in said subject. In addition, since A_{2A}R are also expressed in arteries outside the coronary area, including brain (Murphy *et al.,* 2003) and aorta (Iwamotot *et al.,* 1994), it is likely that A_{2A}R expression abnormalities can be found in obliterative arteriopathy.

Accordingly, the present invention relates to an *in vitro* method of diagnosis or prognosis of an obliterative arteriopathy, particularly coronary artery disease or peripheral obliterative arteriopathy, preferably coronary artery disease, in a subject, comprising the following steps:
a) determining the expression level of adenosine A_{2A} receptors (A_{2A}R) expressed at the cell surface of peripheral blood mononuclear cells (PBMC) obtained from said subject, and
b) comparing the expression level determined in step a) with expression levels of A_{2A}R expressed at the cell surface of PBMC previously obtained from said subject or with the standard expression level of A_{2A}R expressed at the cell surface of PBMC;
an expression level determined in step a) lower than said expression level of A_{2A}R previously obtained from said subject or with said standard expression level of A_{2A}R constituting a marker of an obliterative arteriopathy, particularly a coronary artery disease or peripheral obliterative arteriopathy or of a predisposition to an obliterative arteriopathy, particularly coronary artery disease or peripheral obliterative arteriopathy.

A "subject" refers to a mammal, preferably a human.

An obliterative artheriopathy refers to a pathology of arteries characterized by stenosis leading to obstruction of arteries. Said obstruction may be due to atherosclerosis or thrombosis. Obliterative arteriopathies may affect any kind of arteries, such as brain artery (leading to stroke), coronary artery, lower limb artery. The obliterative arteriopathy is in particular coronary artery disease or peripheral obliterative arteriopathy, preferably coronary artery disease.

Coronary artery disease (CAD) refers to a pathology of the coronary (heart) arteries, such as obstructive coronary artery disease.

Peripheral obliterative arteriopathy includes a stenosis in a vascular bed other than coronary arteries such as lower limb ischaemia and stroke.

Preferably, the peripheral blood mononuclear cells (PBMC) are lymphocyte cells.

In a preferred embodiment of the method of the present invention, the PBMC are obtained from a blood sample collected from brachial vein from said subject. This blood sample can be collected in a tube containing sodium citrate, a polyester gel, and a density gradient liquid.

Methods for determining the expression level of A_{2A}R expressed at the cell surface of PBMC are known in the art (Franceschi *et al.,* 2013; Jacquin *et al.,* 2013).

In an advantageous embodiment of the method of the present invention, the expression level of A_{2A}R is determined by Western blotting.

In another advantageous embodiment of the method of the present invention, A_{2A}R expression level can be determined by flow cytometric assay using intact (not lysed) PBMC samples and an A_{2A}R Ab (see for example Koshiba *et al.,* 1999).

Methods for preparing and lysing PBMC are well known in the art. Commercial kits are available to prepare the PBMC prior to lysis. By way of example, PBMC can be directly prepared using the buffer sample for Western blotting application: 62.5 mM Tris-HCl buffer, pH 8.3, containing 2% SDS, 10% glycerol, 0.01% bromophenol blue and 5% mercaptoethanol. PBMC can be lysed by applying a 10 min sonication treatment at 47 kHz.

A_{2A}R expression level in lysates can be determined by Western blotting using an A_{2A}R antibody (A_{2A}R Ab).

After sonication, samples can be submitted to standard electrophoresis. Separated proteins can be electrotransferred onto a membrane that is then placed in a blot holder of a protein detection device, and incubated with an A_{2A}R Ab.

Blots can be visualized by labeled anti-A_{2A}R Ab species. Bands (45 kDa for the human A_{2A}R) can be densitometrically measured. Results can be expressed as arbitrary units defined as pixels of the A_{2A}R band versus blot background.

As used herein, the term "antibody" encompasses polyclonal or monoclonal antibodies, natural, synthetic or recombinant antibodies, chimeric antibodies such as a humanized antibodies, and the fragments thereof (*e.g.* Fab'₂, Fab, Fv, scFv) having retained their ability to bind A_{2A}R. A full length antibody can be one of five major classes: IgA, IgD, IgE, IgG, and IgM. Several of these full length antibodies are further divided into subclasses or isotypes, such as IgG1, IgG2, IgG3, IgG4, and the like. The subunit structures and three-dimensional configurations of the different classes of immunoglobulins are well known in the art. IgG and/or IgM are the preferred classes of antibodies employed in this invention because they are the most common antibodies in the physiological situation and because they are most easily made in a laboratory setting. The antibodies also include immunoadhesins, diabodies, nanobodies, Janusins, minibodies. Fragments antibodies having retained their ability to bind A_{2A}R include Fab, Fab'2, Fv, scFv fragments.

The term "recombinant antibody" is intended to mean an antibody produced by genetic engineering, for example by cloning and gene amplification.

The term "synthetic antibody" is intended to mean an antibody produced by enzymatic and/or chemical synthesis.

The term "chimeric antibody" is intended to mean an antibody from a particular animal species or a particular class of antibodies, which includes all or part of a heavy chain and/or light chain of an antibody from another species or another antibody class.

The term "chimeric antibody" may also designate a multispecific antibody, *i.e.,* specific to at least two different epitopes. The multispecific antibodies can be obtained from said antibody fragments as defined above.

The term "humanized antibody" refers to a human immunoglobulin in which the residues of the CDRs (Complementary-Determining Region) that form the binding site to the antigen are replaced by those of a non-human monoclonal antibody with a desired specificity, affinity and/or activity.

The antibodies and antibody fragments can be prepared by conventional techniques well known to a person skilled in the art.

A_{2A}R antibodies are known in the art. By way of example, one can use a monoclonal Ab directed to the intracellular domain of A_{2A}R (Koshiba *et al.* 1999) or a commercially available polyclonal A_{2A}R Ab such as REFS SAB1408884 and SAB1410228 (SIGMA-ALDRICH).

Advantageously, the A_{2A}R Ab is the monoclonal antibody produced by the hybridoma deposited under the Budapest Treaty at CNCM, 25 rue du Docteur Roux, 75724 Paris Cedex 15, on November 7, 2014, under the number I-4908. The properties of this monoclonal antibody, named Adonis, are described in By *et al.,* 2009.

Usually, the standard expression level of A_{2A}R at the cell surface of PBMC in a healthy human (*i.e.,* not having coronary artery disease or peripheral obliterative arteriopathy), expressed as arbitrary units, is 1.5 to 2 folds higher than those found in CAD patients.

In a preferred embodiment of the method of the invention, it further comprises a step of determining the presence or the absence of spare A_{2A}R on PBMC, preferably lymphocytes, obtained from said subject, the presence of spare A_{2A}R constituting a further marker of an obliterative arteriopathy, particularly coronary artery disease or peripheral obliterative arteriopathy, preferably coronary artery disease or of a predisposition to an obliterative arteriopathy, particularly coronary artery disease or peripheral obliterative arteriopathy, preferably coronary artery disease.

From a pharmacological point of view, a given ligand can exert maximum biological effect while occupying only a small amount of the available receptors. Such a reserve of receptors, also called spare receptors, may allow for a response to low, transient ligand concentrations and low-affinity interactions which are compatible with several T-cells properties, hence supporting the presence of receptors of reserve for T-cell receptor activation (McNeil *et al.,* 2002). In a practical way, the presence of spare receptors is determined when the half-maximal effective concentration (EC₅₀) that refers to the ligand concentration necessary to reach 50% of the maximal biological effects (cAMP production by PBMC in the present case) is lower than the dissociation constant (*K*_{D}) that refers to the ligand concentration necessary to reach 50% of the maximal binding.

Methods for determining the presence or absence of spare A_{2A}R are known in the art (*e.g.,* see Shryock *et al.,* 1998). Jacquin *et al.* (2013) and Franceschi *et al.* (2013) describe a method of determining the presence or the absence of spare A_{2A}R on PBMC.

Advantageously, the presence or absence of spare A_{2A}R can be determined by using an A_{2A}R irreversible agonist as a specific effector for cAMP production and to determine both the EC₅₀ and K_{D} for A_{2A}R.

An irreversible agonist refers to an A_{2A}R agonist that steadily binds to an extracellular part of the A_{2A}R at least during the time of incubation required to determine both K_{D} and EC₅₀ values (Jacquin *et al.,* 2013; Franceschi *et al.,* 2013).

By way of example, A_{2A}R agonist binding curves can be established using PBMC incubated with six concentrations of A_{2A}R agonist. Non linear regression analysis can be performed with a one site specific binding equation *Y* = *B*max × *X* /(*K*_{D} + *X*) using an appropriate software. *B*max expressed using the same units as *Y* values and *K*_{D} expressed using the same units as *X* values can be estimated for the A_{2A}R agonist binding to each PBMC preparation. Cellular cAMP measurement can be achieved on PBMC incubated with an A_{2A}R agonist (preferably with an agonist-like antibody directed to A_{2A}R, such as the monoclonal antibody Adonis [see below], by a commercially available ELISA kit). Stimulation can be stopped by adding an appropriate compound, such as dodecyltrimethylammonium bromide acetate buffer. Results can be expressed as percent of maximal cAMP production. The results obtained using six concentrations of the A_{2A}R agonist can be examined with an appropriate software and the sigmoidal dose-response curve: *Y* = Bottom + (Top - Bottom)/(1 + 10[(Log EC50 - *X*) × Hill Slope]). Top (*E*max here) and Bottom are plateau values in the units of *Y* and EC50 is the concentration of the A_{2A}R agonist that gives a response half way between Top and Bottom values. Hill slope describes the steepness of the family of curves.

The A_{2A}R agonist can be an agonist-like antibody directed to A_{2A}R, such as the monoclonal antibody Adonis, produced by the hybridoma deposited at CNCM under number I-4908. The monoclonal A_{2A}R Ab named Adonis according to the present invention (see below) is assimilated to an irreversible agonist at least during the time of incubation required to evaluate both K_{D} and EC₅₀ values.

The term "agonist-like antibody" refers to an antibody having the property to activate the A_{2A}R like a conventional organic agonist defined as a molecule that binds to A_{2A}R leading to cAMP production by target cells.

The presence or absence of spare A_{2A}R can also be determined by using an organic antagonist (such as FSPTP) of A_{2A}R to inactivate receptors and reduce the response to an agonist (in particular an irreversible agonist) for determination of equilibrium dissociation constant and EC₅₀ (Shryock *et al.,* 1998).

In another preferred embodiment of the method of the invention, it further comprises a step of determining the adenosine plasma concentration (APC) change during exercise stress testing (EST) in said subject from blood samples, an increase in APC during EST constituting a further marker of an obliterative arteriopathy, particularly coronary artery disease or peripheral obliterative arteriopathy, preferably coronary artery disease or of a predisposition to an obliterative arteriopathy, particularly coronary artery disease or peripheral obliterative arteriopathy, preferably coronary artery disease.

In a particular embodiment, the increase in APC is associated with a positive EST.

A positive EST refers to the existence of a significant ST segment wave changes (>2mm) and ST segment horizontal or down looping depression and/or a characteristic chest pain).

This step can be carried out in combination or not with the step of determining the presence or the absence of spare A_{2A}R on PBMC as described above.

If this step is carried out, the PBMC used for determining the expression level of A_{2A}R and optionally for determining the presence or absence of spare A_{2A}R is obtained from said subject at basal level (*i.e.,* before the exercise stress test).

Methods for determining APC in a subject from a blood sample are well known in the art (Guieu *et al.,* 1999; Saadjian *et al.,* 2002 and 2009; Jacquin *et al.,* 2013).

By way of example, after collection, the blood sample is centrifuged and then deproteinized. Adenosine is then quantified using high-pressure liquid chromatography.

EST is well known in the art since it is a widely used method of CAD screening (Banerjee *et al.,* 2012). It includes running exercise, cycling exercise, or cycling exercise with arms for lower limbs paralyzed patients.

Advantageously, the EST is a treadmill exercise test that can be carried out according to the Bruce protocol (Bruce, 1974). During this procedure workload is progressively increased from 20 watts every min on a bicycle, until the maximal hear rate (220 beats/min-age in year). It is also possible to evaluate during exercise stress test the VO₂ max, using air way mask to evaluate O₂ consumption.

The APC change is advantageously determined by measuring APC at basal level (T0) and at the end of EST (T1).

The present invention also relates to the hybridoma producing an agonist-like monoclonal antibody directed to the human A_{2A}R (named Adonis) deposited at CNCM on November 7, 2014 under the number I-4908.

The present invention also relates to the agonist-like monoclonal antibody directed to the human A_{2A}R produced by the hybridoma cell line deposited on November 7, 2014 at CNCM under the number I-4908, or a fragment thereof.

The present invention also relates to an isolated fragment from said agonist-like monoclonal antibody produced by the hybridoma deposited at CNCM under the number I-4908, consisting of one complementary determining region (CDR) or comprising one, two or the three CDRs, preferably the three CDRs, from said agonist-like monoclonal antibody.

Methods for determining the CDRs in an antibody are well known to a person skilled in the art (Kuroda *et al.,* 2012).

Said isolated fragment can be a Fab or Fab'2 fragment.

The present invention also relates to a polypeptide comprising one, two or the three CDRs, preferably the three CDRs, from said agonist-like monoclonal antibody produced by the hybridoma deposited at CNCM under the number I-4908.

Advantageously, said isolated fragment or polypeptide is able to bind A_{2A}R.

The present invention also relates to an agonist-like monoclonal antibody directed to the human A_{2A}R, or a fragment thereof, comprising the three CDRs from said agonist-like monoclonal antibody produced by the hybridoma deposited at CNCM under the number I-4908.

The present invention also relates to the use of an agonist-like monoclonal antibody directed to the human A_{2A}R or an isolated fragment or a polypeptide able to bind A_{2A}R according to the invention for the *in vitro* diagnosis or prognosis of an obliterative arteriopathy, particularly coronary artery disease or peripheral obliterative arteriopathy, preferably coronary artery disease, in a subject.

Advantageously, said agonist-like monoclonal antibody directed to the human A_{2A}R is produced by the hybridoma deposited at CNCM under the number I-4908.

The present invention also relates to a kit for the *in vitro* diagnosis or prognosis of an obliterative arteriopathy, particularly coronary artery disease or peripheral obliterative arteriopathy, preferably coronary artery disease, in a subject comprising:
- an agonist-like monoclonal antibody directed to the human A_{2A}R, such as the monoclonal antibody produced by the hybridoma deposited at CNCM under the number I-4908, or an isolated fragment or a polypeptide able to bind A_{2A}R according to the invention,
- at least one positive control, such as PBMC lysate from a subject having an obliterative arteriopathy, particularly coronary artery disease or peripheral obliterative arteriopathy, preferably coronary artery disease, and
- at least one negative control, such as PBMC lysate from an healthy subject.

In addition to the above features, the invention further comprises other features which will emerge from the following description, which refers to an example illustrating the present invention, as well as to the appended figures.
**Figure 1** shows the adenosine plasma concentration (APC) at basal level (T0), at the end of exercise stress testing (T1), and after 10 min recovery (T2) in 30 patients. a: p<0.01 compared with T0.
**Figure 2** shows the APC at basal level (T0), at the end of EST (T1), and after 10 min recovery (T2) in patients with positive (+, n=15) or negative (-, n=15) EST and in 15 healthy subjects who served as controls. a: p<0.01 compared with T0+; b: p=0.12 compared with TO-.
**Figure 3** shows **(A)** the A_{2A} adenosine receptor expression (A_{2A}R) evaluated by Western blotting in patients (whole population of patients n= 30) who underwent an EST as scheduled of their clinical follow-up and in 15 healthy subjects who served as controls. EST was positive in 15 patients and negative in 15 patients. A_{2A}R expression as a function of positive or negative EST and in controls is also given. **(B):** example of Western blot of A_{2A}R obtained from a patient with positive EST (EST+), negative EST (EST-) and in control subject.

### EXAMPLE: DIAGNOSIS OF CORONARY ARTERY DISEASE

### 1) Patients and Methods

### Patients

It was performed a monocenter observational study including 33 consecutive patients: 25 men and 8 women, mean age 62.6±13.5 years, range 33-89. Among the patients, 16 had established coronary artery disease and underwent EST as part of their scheduled clinical follow-up and 17 underwent EST for the first time as part of their clinical investigations. Fifteen healthy subjects (9 men and 6 women, mean age 60± 15.6 years, range 27-81) served as controls for basal biological parameters.

### EST procedure

All patients performed the treadmill exercise test according to the Bruce protocol (Bruce RA 1974). The EST was considered positive when the patient developed significant ST segment T wave changes (>2 mm) and ST segment horizontal or down looping depression and or typical chest pain.

### APC measurement

Blood samples (3 mL) were collected (obtained) and processed as described previously (Saadjian *et al.,* 2002; Deharo *et al.,* 2012), using laboratory-made tubes containing 2 mL of cold-stop solution under vacuum. The stop solution was composed of saline containing dipyridamole 0.2mM; ethylene diamine tetracetic acid disodium (Na 2 EDTA 4 mM); erythro-9-(2-hydroxy-3-nonyl adenine (EHNA, 5mM); alpha, beta-methyleneadenosine 5' diphosphate (AOPCP, 79 mM); 2' deoxycoformycin 10 µg/mL; and heparin sulfate 1IU/mL.

This method allows whole blood to mix quickly with the stop solution in order to prevent red blood cell uptake and degradation of adenosine (Guieu *et al.,* 1999, Saadjian *et al.,* 2002, Deharo *et al.,* 2012, Bonello *et al.,* 2013). For patients, samples were collected at basal level, at the end of EST, and after 10 min recovery period.

APC dosage has been described (Guieu *et al.,* 1999; Saadjian *et al.,* 2002). In brief, after collection, samples were quickly centrifuged (4°C, 1500g) and then deproteinized (perchloric acid, 70 % 1/10V). After another centrifugation (2500 g for 10 min) supernatants were pipetted off, mixed with 1mL of phosphate buffer and analyzed by HPLC. A modular system with a diode array detector (Chrom System, Germany) was used. Samples were eluted with a methanol gradient (0% to 35% in 60 min) on a Merck LiChrospher C18 column (Nottingham, UK). Adenosine was identified using its elution time and spectrum, and quantified by comparison of peak areas with those of known quantities of adenosine. The sensitivity threshold was 2 nM of plasma matrix. The intra- and inter-assay coefficients of variation ranged from 1% to 3%.

### A_{2A}R expression on peripheral blood mononuclear cells

PBMC was chosen because behavior of A_{2A}R expressed at the surface of peripheral blood mononuclear cells (PBMC) mirrors the behavior of these receptors on cells of the cardiovascular system (Varani *et al.,* 2003). The methodology has been described (Franceschi *et al.,* 2013; Jacquin *et al.,* 2013). In brief, blood samples from brachial vein of patients were collected at basal level into tubes containing sodium citrate, a polyester gel, and a density gradient liquid (BD Vacutainer CPT, Beckton Dickinson, Franklin Lakes, NJ). PBMC were prepared according to the manufacturer's instructions prior to lysis using 62.5 mM Tris-HCl buffer, pH 8.3, containing 2% SDS, 10% glycerol, 0.01% bromophenol blue and 5% mercaptoethanol followed by 10 min sonication treatment at 47 kHz. A_{2A}R expression in lysates was determined by Western blotting using the monoclonal antibody directed to A_{2A}R named Adonis (By *et al.,* 2009). After sonication, samples were submitted to standard electrophoresis in Mini Protean II system (BioRad, Hercules, CA). Separated proteins in 12% acrylamide minigel were electrotransferred onto a PVDF membrane that was then placed in the blot holder of the SNAP i.d. protein detection system (Millipore, Billerica, MA), saturated with non-fat dried milk, and incubated 20 min with Adonis (1 µg/ml). Blots were visualized by horseradish peroxidase-labeled anti-mouse antibodies and enhanced chemiluminescence substrate (SuperSignal West Femto, Pierce Biotechnology, Rockford, IL) using a Kodak system. Bands (45 kDa for A_{2A}R) were measured densitometrically using Image J 1.42q software developed at the U.S. National Institutes of Health. Results were expressed as arbitrary units defined as pixels of the A_{2A}R band versus blot background.

### Statistical Analysis

Quantitative variables were expressed using medians and interquartile or means and standard deviations (SD). Variance analysis (two-way ANOVA) was used to compare APC and A_{2A}R expression. A Wilcoxon test was used for pair-wise comparisons with Bonferroni correction to account for the inflation in type I error. All statistical tests were two-sided and P-values less than 0.05 were considered statistically significant. Analyses were performed with Prism® software.

### 2) Results

Clinical characteristics of the patients are given in Table 1 below. Results of exercise stress testing are given in Table 2 below.

**Table I: Patient baseline characteristics n=30. CABG: Coronary artery bypass graft; CAD: Coronary artery disease; PCI: Percutaneous coronary intervention**

| | |
|---|---|
| Demographics | |
| Age years (mean, range) | 62.6[33-89] |
| Men/women | 22/8 |
| Cardiac Risk Factors | |
| Diabetes | N=8 |
| Current or former smoker | N=12 |
| Hyperlipidemia | N=13 |
| Cardiac disease | |
| Documented CAD | N=16 |
| Prior MI | None |
| Prior PCI | N=14 |
| Prior CABG | N=0 |

**Table 2: Stress test results. Data are presented as median and interquartile range.**

| | |
|---|---|
| EST Performance | |
| Duration (min) | 6.4 [6.2-8.8] |
| Maximum Workload (Mets) | 120 [75-135] |
| Peak HR (b.p.m) | 141 [125-144] |
| Peak SBP (mmHg) | 194 [185-211] |
| Chest Pain | |
| No | N=27 |
| Yes | N=3 |

Fifteen EST were positive and fifteen were negative, one was equivocal and was excluded from further investigations. One patient was unable to reach maximal heart rate.

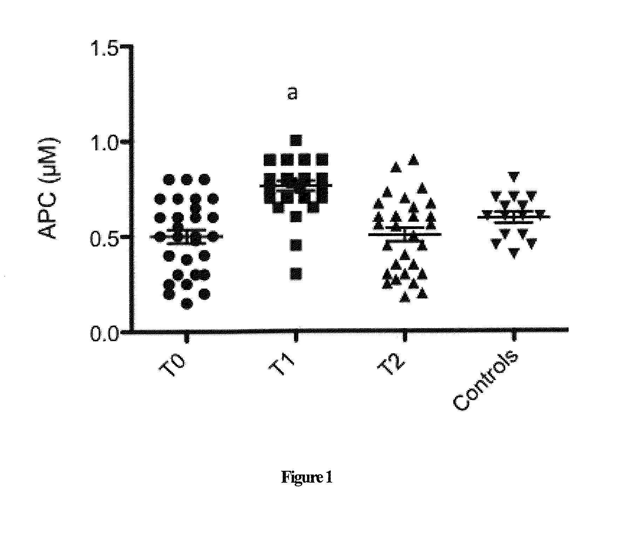

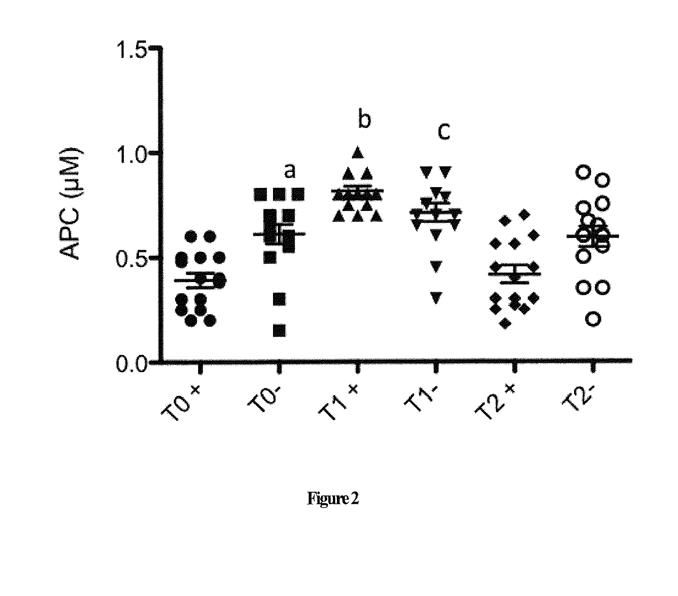

APC (µM) (median [25th-75th percentile]) for the whole population of patients (n=30) was 0.6 [0.5-0.7] at basal level, which was not significantly different from APC of controls: 0.6 [0.5-0.7]; p=0.7. Basal APC of EST- or EST + patients did not differ significantly from controls: EST -: 0.65 [0.55-0.7] vs controls; p=0.7, EST +: 0.6[0.5-0.6] vs controls p= 0.7; and see figure 2. During EST, APC increased significantly in the whole population of patients, 0.6 [0.5-0.7] vs 0.8 [0.7-0.9], p=0.0002. However, this increase was limited to EST+ patients, 0.6[0.5-0.6] vs 0.80 [0.75-0.9]; p<0.01. APC did not increase significantly in the EST- group 0.65 [0.55-0.7] vs 0.70 [0.65-0.9]; p=0.12.; see Figure 2. APC returned to basal values after 10 min recovery, 0.52 [0.31-0.63] and see Figure 1.

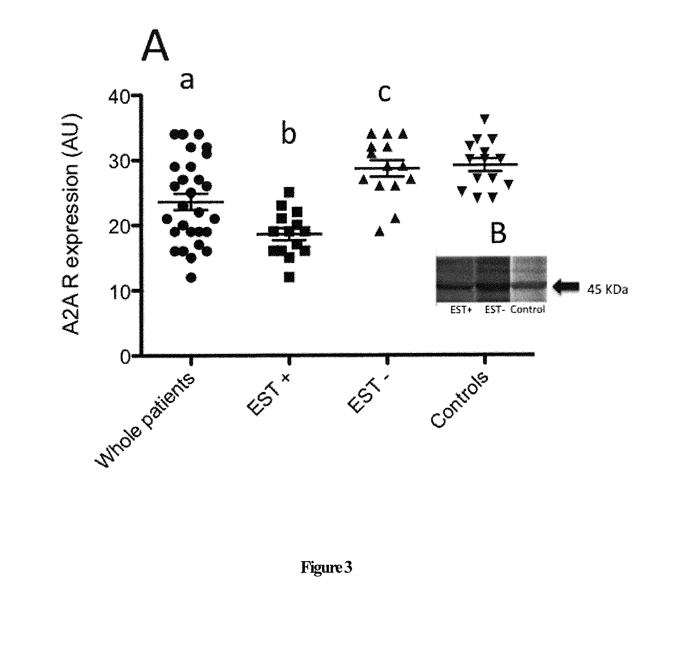

A_{2A}R expression (arbitrary units, AU) was lower than controls in the whole population of patients, 22.5 [19-29] vs 30 [26.75-32.25]; p=0.005 (see Figure 3). However, the low A_{2A}R expression was limited to EST+ patients, 19 [16-21.25]; p<0.0001 compared with controls). A_{2A}R expression of EST- patients was not significantly different from controls, 29 [26-32.25]; p=0.75 and see Figure 3.

### 3) Conclusion

These results show that patients with a positive treadmill test had lower A_{2A}R expression compared with negative treadmill test and a significant increase in APC at peak exercise compared to patients with a negative treadmill test. Such an increase is likely related to a myocardial ischemia process that occurs during EST in patients with obstructive coronary artery disease.

The present study suggests low basal A_{2A}R expression and increase in APC during treadmill is associated with positive EST and coronary artery disease.

Since 100% of the patients having a CAD also have low basal A_{2A}R expression, the present study suggests that low A_{2A}R expression level of mononuclear cells is associated with CAD. The measure of basal A_{2A}R expression of mononuclear cells can therefore be useful to screen CAD patients.

### REFERENCES

Banerjee A, et al., Int J Clin Pract. 2012; 66:477-92.
Berwick ZC, et al., J Mol Cell Cardiol. 2012; 52:912-9.
Bonello L, et al., J Am Coll Cardiol. 2014; 63:872-7.
Bruce RA. Listing Res Cardiovascular Field. 1964; 10:283-6. Review.
Bruce RA. Methods of exercise testing. Step test, bicycle, treadmill, isometrics. Am J Cardiol. 1974 May 20;33(6):715-20.By Y, et al., Mol Immunol. 2009; 46:400-5.Deharo JC, et al., Heart. 2012; 98:855-9.
Eckle T, et al., Circulation. 2007; 115:1581-90.
Franceschi F, et al., Europace. 2013; 15:1328-32.
Grenz A, et al., Antioxid Redox Signal. 2011; 15:2221-34.
Guieu R, et al., J Liq Chromatogr. 1999; 22:1829-1841.
Hedegaard ER, et al., Eur J Pharmacol. 2014; 723:216-26.
Iwamoto T et al. Biochem Biophys Res Commun. 1994; 199:905-10.
Jacquin L, et al., FEBS Open Bio. 2013; 3:1-5.
Koshiba M et al., Mol Pharmacol. 1999; 55:614-24.
Kuroda D, et al., Protein Eng Des Sel. 2012; 25:507-21
McNeil LK, et al., Proc. Natl. Acad. Sci. USA. 2002; 99:4520-4525.
Murphy K et al. Mol Pharmacol. 2003; 64:640-9.
Nyman I, International Journal of Cardiology. 1993; 39:131-42.
Olsson RA, et al., Physiol Rev. 1990; 70:761-845.
Paganelli F, et al., Eur J Clin Invest. 2000; 30:105-10.
Pinto-Duarte A, et al., J Neurochem. 2005; 93:595-604.
Saadjian AY, et al,, Circulation. 2002; 106:569-74.
Saadjian AY, et al., Eur Heart J. 2009; 30:1510-5.
Sanjani MS, et al., Am J Physiol Heart Circ Physiol. 2011; 301:H2322-33.
Shryock JC, et al., Am J Cardiol. 1997; 79:2-10. Review.
Shryock JC, et al., Circulation. 1998; 98:711-8.
Sollevi A. et al., Prog Neurobiol. 1986; 27:319-49. Review.
Tang Z, et al., Clin Pharmacol Ther. 2007; 82:435-40.
Varani K, et al., FASEB J. 2003; 17:280-2.

## Claims

1. An *in vitro* method of diagnosis or prognosis of an obliterative arteriopathy in a subject, **characterised in that** it comprises the following steps:
a) determining the expression level of adenosine A_{2A} receptors (A_{2A}R) expressed at the cell surface of peripheral blood mononuclear cells (PBMC) obtained from said subject, and
b) comparing the expression level determined in step a) with expression levels of A_{2A}R expressed at the cell surface of PBMC previously obtained from said subject or with the standard expression level of A_{2A}R expressed at the cell surface of PBMC,
an expression level determined in step a) lower than said expression level of A_{2A}R expressed previously obtained from said subject or with said standard expression level of A_{2A}R constituting a marker of an obliterative arteriopathy or of a predisposition to an obliterative arteriopathy.

2. The method according to claim 1, **characterised in that** the obliterative arteriopathy is selected from the group consisting of coronary artery disease and peripheral obliterative arteriopathy, preferably coronary artery disease.

3. The method according to claim 1 or claim 2, **characterised in that** the PBMC are obtained from a blood sample collected from brachial vein of said subject.

4. The method according to any one of claims 1 to 3, **characterised in that** the expression level of A_{2A}R is determined by Western blotting or flow cytometric assay.

5. The method according to any one of claims 1 to 4, **characterised in that** it further comprises a step of determining the presence or the absence of spare adenosine A_{2A} receptor (spare A_{2A}R) on PBMC obtained from said subject, the presence of spare A_{2A}R constituting a further marker of an obliterative arteriopathy or of a predisposition to an obliterative arteriopathy.

6. The method according to claim 4 or claim 5, **characterised in that** the expression level of A_{2A}R and/or the presence of spare A_{2A}R are determined with the agonist-like monoclonal antibody produced by the hybridoma deposited at CNCM under number I-4908.

7. The method according to any one of claims 1 to 6, **characterised in that** it further comprises a step of determining the adenosine plasma concentration (APC) change during exercise stress testing (EST) in said subject from blood samples, an increase in APC during EST constituting a further marker of an obliterative arteriopathy or a predisposition to an obliterative arteriopathy.

8. The method according to claim 7, **characterised in that** the exercise stress testing is a treadmill exercise test.

9. The hybridoma producing an agonist-like monoclonal antibody directed to the human adenosine A_{2A} receptor deposited at CNCM on November 7, 2014, under the number I-4908.

10. The agonist-like monoclonal antibody directed to the human adenosine A_{2A} receptor produced by the hybridoma of claim 9, or a fragment thereof.

11. An isolated fragment from the antibody of claim 10, consisting of one complementary determining region (CDR) or comprising one, two or the three CDRs, from said agonist-like monoclonal antibody.

12. A polypeptide comprising one, two or the three CDRs from the antibody of claim 10.

13. Use of an agonist-like monoclonal antibody directed to the human adenosine A_{2A} receptor or an isolated fragment of claim 11 a polypeptide of claim 12 wherein said isolated fragment and polypeptide is able to bind A_{2A}R, for the *in vitro* diagnosis or prognosis of an obliterative arteriopathy in a subject.

14. Use according to claim 13, **characterised in that** the agonist-like monoclonal antibody directed to the human adenosine A_{2A} receptor is the antibody of claim 10.

15. Kit for the *in vitro* diagnosis or prognosis of coronary artery disease or peripheral obliterative arteriopathy in a subject comprising:
- an agonist-like monoclonal antibody directed to the human adenosine A_{2A} receptor or an isolated fragment of claim 11 or a polypeptide of claim 12 wherein said isolated fragment and polypeptide is able to bind A_{2A}R,
- at least one positive control, and
- at least one negative control.
